# EUROPEAN PATENT SPECIFICATION

(11) **EP 2 156 254 B1**
(45) Date of publication and mention of the grant of the patent: **06.12.2017**
(21) Application number: 08747747.7
(22) Date of filing: 07.05.2008
(51) Int. Cl.: G05B 23/02, G06Q 10/00, G06Q 50/22, G06Q 50/04

(54) **HEALTH MANAGEMENT OF SINGLE OR MULTI-PLATFORM SYSTEMS**
INTEGRITÄTSVERWALTUNG VON EINZEL- ODER MEHRPLATTFORMSYSTEMEN
GESTION DE DONNÉES DE SANTÉ POUR SYSTÈMES À UNE OU PLUSIEURS PLATE-FORMES.

(30) Priority: 07.05.2007 US 745258
(43) Date of publication of application: 24.02.2010
(73) Proprietor: The Boeing Company, Chicago, IL 60606-1596 (US)
(72) Inventor: VIAN, John L., Renton, Washington 98056 (US); CLARK, Gregory J., Seattle, Washington 98115 (US)
(74) Representative: Howson, Richard G.B.
(86) International application number: PCT/US2008/062833
(87) International publication number: WO 2008/154097

(56) References cited:
- EP-A- 1 455 313
- EP-A- 1 521 152
- US-A1- 2004 020 994
- US-A1- 2006 064 291

## Description

### BACKGROUND

Embodiments of the invention relate generally to systems and methods for health data management, including health management systems and methods for health data handling and prognostic reasoning for multiple types of systems.

Modern health management of complex machines and systems, including complex aerospace systems, may go beyond simply monitoring operating conditions. Health management may also include assimilation of available information for determination of predicted failure modes and failure times, possible corrective actions, and planning and scheduling options. Thus, health management may provide a number of interconnected and cooperative functions to comprehensively manage the health of the system.

Although prior art systems and methods have achieved desirable results, there is room for improvement. For example, conventional health management systems for aircraft are typically highly-individualized, employing customized frameworks, individualized prognostic algorithms, and dissimilar data management and storage techniques. The current lack of commonality among various health management systems inhibits the ability to merge various types of onboard and offboard generated data across the particular aircraft, or across groupings of aircraft (e.g. squadron or fleet of aircraft assigned to a particular flight route). Novel systems and methods that mitigate these negative characteristics of the prior art would therefore have utility. EP1521152 describes a system and method for dynamic multi-objective optimization of machine selection, integration and utilization.

### SUMMARY

Embodiments of systems and methods in accordance with the present disclosure are directed to health data management, including health data management for multi-platform systems. Such embodiments may advantageously increase aircraft system reliability, safety, maintainability, availability, and affordability resulting in improved mission performance and operational capabilities.

In an aspect, a method of monitoring health information for a multi-platform system is provided as defined in claim 1.

In an aspect, a system for monitoring health information for a multi-platform system is provided as defined in claim 6.

In an aspect, one or more computer-readable media containing computer-readable instructions are provided as defined in claim 11.

In one example, a method of monitoring health information for a multi-platform system includes receiving health information from one or more subsystems of a plurality of platforms, and analyzing the health information using one or more reasoner algorithms configured to predict a potential failure of the one or more subsystems. Upon prediction of a potential failure, the method includes providing a recommended action and prognostic characteristic of the one or more subsystems. In alternate examples, the method may further include translating at least some of the health information for each of the one or more subsystems into a common format, and storing the translated health information into a database for subsequent analysis.

In further examples, the plurality of platforms may include one or more flight vehicles, and analyzing the health information may include retrieving the translated health information from the database, and analyzing the translated health information using one or more ground-based reasoner algorithms. Alternately, providing a prognostic characteristic may include providing at least one of a repair order, a replacement order, and a maintenance order.

In another example, a system for monitoring health information for a multi-platform system includes three components. A first component is configured to receive health information from one or more subsystems of a plurality of platforms. A second component is configured to analyze the health information using one or more reasoner algorithms configured to at least one of diagnose and predict a potential failure of the one or more subsystems. A third component is configured to, upon diagnosis of a failure, provide recommended action, and upon prediction of a potential failure, provide a prognostic characteristic of the one or more subsystems.

The features, functions, and advantages that have been discussed can be achieved independently in various examples of the present invention or may be combined in yet other examples further details of which can be seen with reference to the following description and drawings.

### BRIEF DESCRIPTION OF THE DRAWINGS

Embodiments of systems and methods in accordance with the present disclosure are described in detail below with reference to the following drawings.
Figure 1 is a schematic view of a method of performing integrated health management of a multi-platform system in accordance with an embodiment of the invention;
Figure 2 is a schematic view of a system for performing multi-platform integrated health management in accordance with an embodiment of the invention;
Figure 3 shows a representative plurality of health data sources for a military aircraft and a commercial aircraft;
Figure 4 is a representative test data collection process for a military aircraft being tested at a test station;
Figure 5 is a representative health data collection process for a commercial aircraft;
Figure 6 is another representative health data collection process for a military aircraft;
Figure 7 is another representative health data collection process for another aircraft;
Figure 8 is still another representative health data collection process for an aircraft; and
Figure 9 shows representative diagnostic reasoning algorithms suitable for use in systems and methods in accordance with alternate embodiments of the invention.

### DETAILED DESCRIPTION

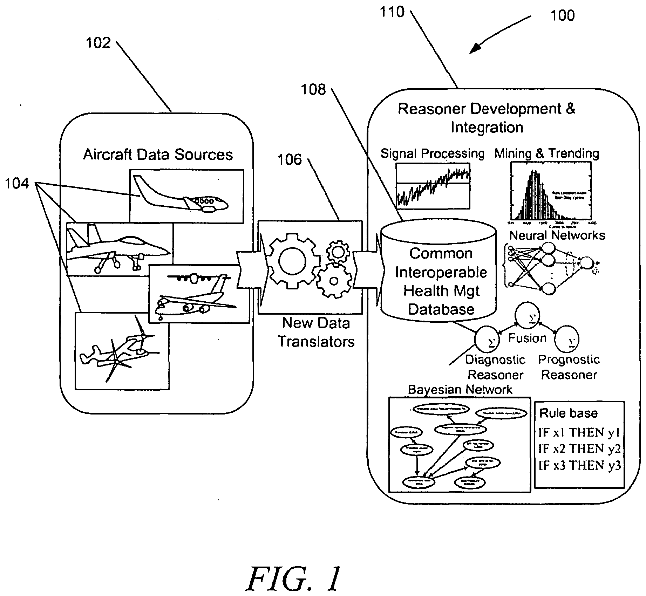

The present disclosure relates to systems and methods for health data management, including health data management for multi-platform systems. Many specific details of certain embodiments are set forth in the following description and in Figures 1-9 to provide a thorough understanding of such embodiments. One skilled in the art, however, will understand that the present invention may have additional embodiments, or that the present invention may be practiced without several of the details described in the following description.

In general, embodiments of systems and methods in accordance with the present disclosure may provide capabilities to translate, store in a common format, view, analyze, merge, and process (via model-based and non-model-based diagnostic and prognostic algorithms) data from multiple aircraft health management data sources. Such systems and methods may enable common algorithms to be re-used for operation on data from multiple aircraft types, and from multiple aircraft health management data sources, including: 1) operational data (*e.g.* parameter data and faults), 2) maintenance data (*e.g.* maintenance actions, part installations and removals, and test stand data), and 3) reference data (*e.g.* flight recorder configuration, software configuration, fault tolerance levels, expected parameters, test stand configuration data, system and subsystem organization, and engineering units).

Figure 1 is a schematic view of a method 100 of performing integrated health management of a multi-platform system in accordance with an embodiment of the invention. The method 100 includes receiving health information at a block 102 from one or more subsystems of a plurality of platforms 104. In the embodiment shown in Figure 1, the platforms 104 are depicted as various types of commercial and military aircraft. However, it will be appreciated that in alternate embodiments, other suitable types of platforms may be monitored. In the event that the health information received from the plurality of platforms is not in a common format, then the health information may be appropriately translated into a common format at a block 106, and may be stored within a common interoperable health management database at a block 108.

As further shown in Figure 1, at a block 110, the health information is analyzed using one or more reasoner algorithms. In some embodiments, the one or more reasoner algorithms are configured to diagnose and/or predict a potential failure of the one or more subsystems, including, for example, failure mode, time of failure, etc. Upon diagnosis or prediction of a potential failure, the analysis performed at block 110 may further provide a diagnostic or prognostic characteristic of the one or more subsystems, such as a repair order, a replacement order, a maintenance order, or any other suitable prognostic characteristics. The analysis performed at block 110 may also include mining and trending analyses, or any other desired analyses.

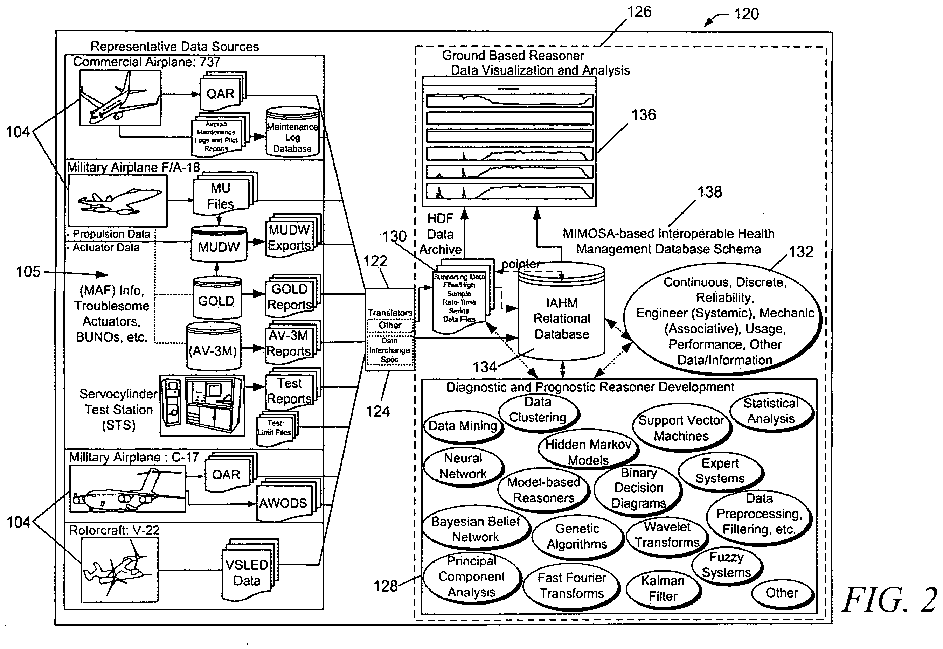

Figure 2 is a schematic view of a system 120 for performing multi-platform integrated health management in accordance with an embodiment of the invention. As noted above, the system 120 includes one or more translators 122 that convert health information received from the plurality of platforms 104 into a common format. As noted above, the platforms 104 may be flight vehicles or any other suitable types of platforms. Alternately, the health information may be provided by maintenance personnel, maintenance systems, and component or subsystem tests 105, such as may be conducted on test stands, laboratories, wind tunnels, or any other suitable test environment. The one or more translators 122 may operate in accordance with a data interchange specification 124 to properly convert the health information into the desired common format.

The system 120 further includes a reasoner module 126. The reasoner module 126 analyzes the health information using one or more reasoner algorithms 128. As noted above, the one or more reasoner algorithms 128 may be configured to diagnose failures, to provide prognostics, to perform data mining and trending analyses, or any other desired types of analyses. The reasoner module 126 may operate on one or more data files 130 that may be accessed by the reasoner algorithms 128 to perform the desired analyses. The one or more data files 130 may be used to store large volumes of high-sample rate (e.g. 100Hz for several flight hours) data that are seldomly used by the reasoner module 126, e.g. for specific event-driven analyses. The data files may be compressed for efficient long-term storage and optimized for fast retrieval of historical data. For example, using Hierarchical Data Format, Version 5 (HDF5), a general-purpose, machine-independent standard for storing scientific data, developed by the National Center for Supercomputing Applications (NCSA). The data files 130 may be linked (via software pointers) to the relational database 134 for use by the reasoner algorithms 128.

As further shown in Figure 2, the reasoner module 126 of the health management system 120 may include a relational database 134. The relational database 134 may be used for storing many types of vehicle health management data (provided by translators 122). It may also include access to high rate data files 130 stored externally to the relational database for infrequent event-driven detailed analyses of specific data parameters. In further embodiments, the relational database 134 may advantageously enable one or more of the following three basic vehicle health management data associations: 1) multiple data set associated from the same data source (*e.g.* aircraft flight data compared from one flight to the next), 2) two different data sources from the same aircraft (*e.g.* aircraft flight data compared to maintenance action data compared to test stand data for a particular part), and 3) multiple different aircraft platforms (*e.g.* airplane to rotorcraft). The reasoner 126 may also include a support database 132. The support database may include empirical or analytically derived information regarding failure modes of one or more subsystems of the various platforms 104, system design specifications, and maintenance informations, and operator knowledge. The reasoner module 126 may also include a data visualization component 136 that enables visual (*e.g.* graphical) analysis of the health information, the relational information from the relational database 134, as well as the results of the analyses performed by the reasoner algorithms 128.

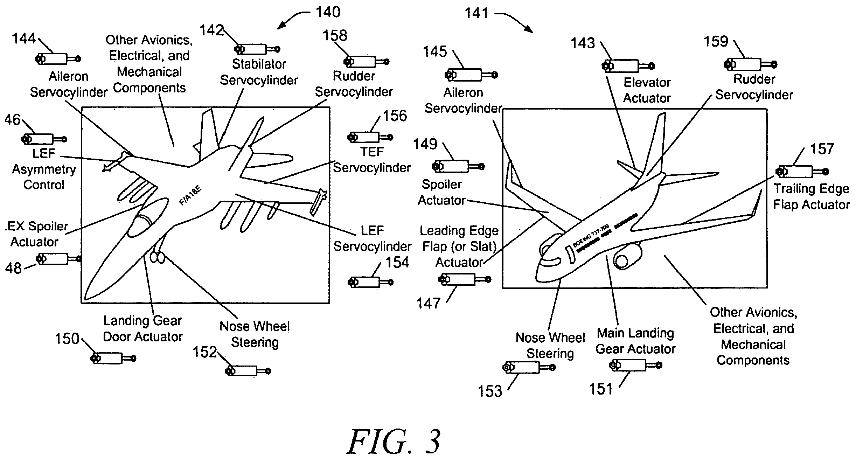

Embodiments of methods and systems in accordance with the present invention may be used for monitoring and managing multi-platform systems having a wide variety of health information sources. For example, Figure 3 shows a plurality of health data sources for a representative military aircraft 140 and a representative commercial aircraft 141. For the military aircraft 140, the plurality of health data sources includes one or more components of a stabilator assembly 142 which provides position control of horizontal tail surfaces, one or more components of an aileron assembly 144 for controlling aileron position, one or more components of an LEF (Leading Edge Flap) assembly 146 for controlling symmetry characteristics, one or more components of an LEX (Leading Edge Extension) assembly 148 for actuating aircraft spoilers, one or more components of a nose landing gear assembly 150, one or more components of a nose wheel steering assembly 152, one or more components of an LEF HDU (Hydraulic Drive Unit) assembly 154, one or more components of a TEF (Trailing Edge Flap) assembly 156, and one or more components of a rudder control assembly 158. Of course, any other desired sources of health data may be used, including avionics systems, engines and other propulsion system components, and any other desired systems and subsystems.

Similarly, for the commercial aircraft 141, the plurality of health data sources includes an elevator actuator 143 for elevator control, an aileron assembly 145 for controlling aileron position, one or more components of an LEF or LES (Leading Edge Slat) assembly 147, a spoiler actuator 149 for actuating aircraft spoilers, one or more components of a landing gear assembly 151, one or more components of a nose wheel steering assembly 153, one or more components of a TEF assembly 157, and one or more components of a rudder control assembly 159. Other avionics, electrical, and mechanical components of the aircraft 141 may also be monitored.

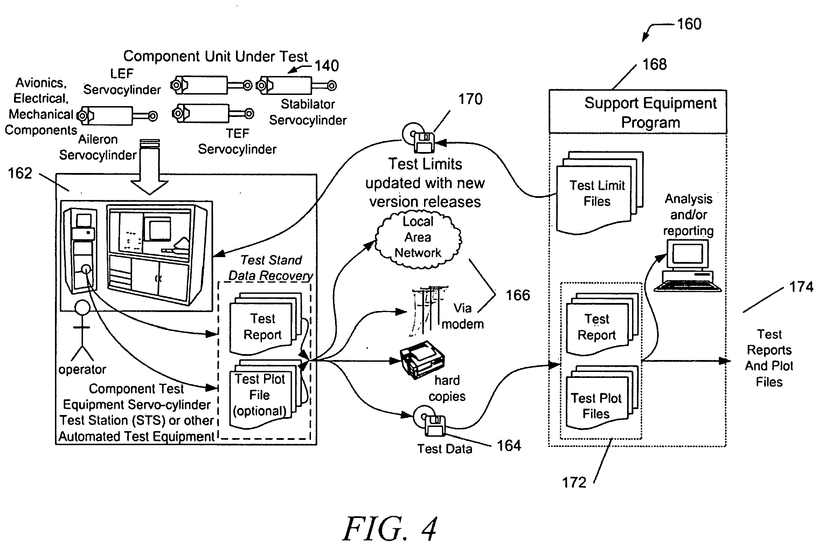

It will be appreciated that embodiments of systems and methods in accordance with the present disclosure may use health information obtained from tests conducted on the systems and subsystems of the various platform types. For example, Figure 4 is a representative test data collection process 160 for a military aircraft 140 being tested at a test station 162. In the embodiment shown in Figure 4, the test station 162 is depicted as a servocylinder test station (STS), which is a hydraulic test station used to perform fully automated performance verification and diagnostic fault isolation on flight control servoactuators and servoactuator subassemblies removed from the military aircraft 140 at intermediate and depot levels. Such test stations are available for various types of aircraft and aircraft components.

The data collection process 160 shown in Figure 4 provides digital data on the results of the tests performed on all health information sources of the aircraft 140, including the flight control actuators described above with respect to Figure 3. A control component 168 may provide a test plan or a prescribed set of test limits 170 to the test station 162 to ensure that the desired health information is acquired during the testing. Test data 164 acquired using the test station 162 may include a summary of the test results, as well as the actual raw data stream captured during the test. These data 164 may be stored on a storage device, or transmitted via a communication network 166 (*e.g.* local area network, global network, modem, wireless link, etc.) for subsequent post-processing and analysis.

The test data 164 are communicated to an analysis module 172, which in this embodiment is shown as part of the control component 168. The analysis module 172 may perform a method of managing health information in accordance with the teachings of the present disclosure, such as the methods described above with respect to Figures 1 and 2. Alternately, the analysis module 172 may perform selected portions of the methods described above, such as translating the test data 164 into a desired format for transmittal and subsequent analysis by the one or more reasoner algorithms. In further embodiments, output data 174 from the control component 168 may remain unprocessed and may be input to a separate system, such as the system 120 described above and shown in Figure 2, for processing and analysis.

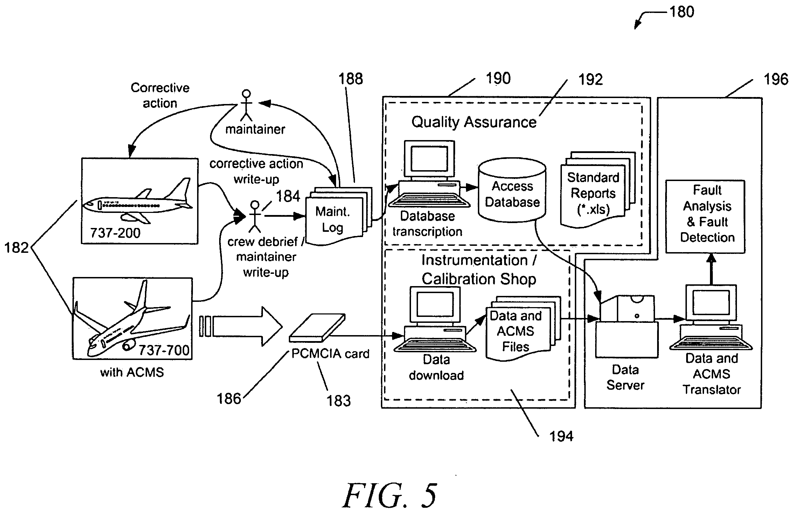

Figure 5 is a representative health data collection process 180 for gathering health information from one or more commercial aircraft 182. In this embodiment, the process 180 includes one or more personnel of an airplane operator (*e.g.* commercial airline company) to support development of the health data 183. More specifically, the health data 183 may include write-ups or reports 184 by airline crew or maintenance personnel, which may be assembled as aircraft maintenance logs 188, as well as cartridge retrieval data 186 from operational aircraft. In some embodiments, data sources and health data for commercial airplanes 182 may include Aircraft Condition Monitoring System (ACMS) reports, Aircraft Maintenance Logs, and Quick Access Recorder (QAR) data. Alternately, health data may be obtained for commercial aircraft from laboratory data collection, and other means appropriate to access health management data that provide a sample of cross-platform data exhibiting a diversity of types (*e.g.* fault codes, processed continuous, discrete, sampled high-bandwidth, context, etc.) and preferably conducive to use by advanced diagnostic reasoners.

As further shown in Figure 5, the health data 183 may be input to a data gathering component 190, which may include a quality assurance portion 192 and an instrumentation and calibration portion 194. The health data 183 are then transmitted to a health data management component 196 which includes systems and performs methods in accordance with the present disclosure, such as the systems and methods described more fully above.

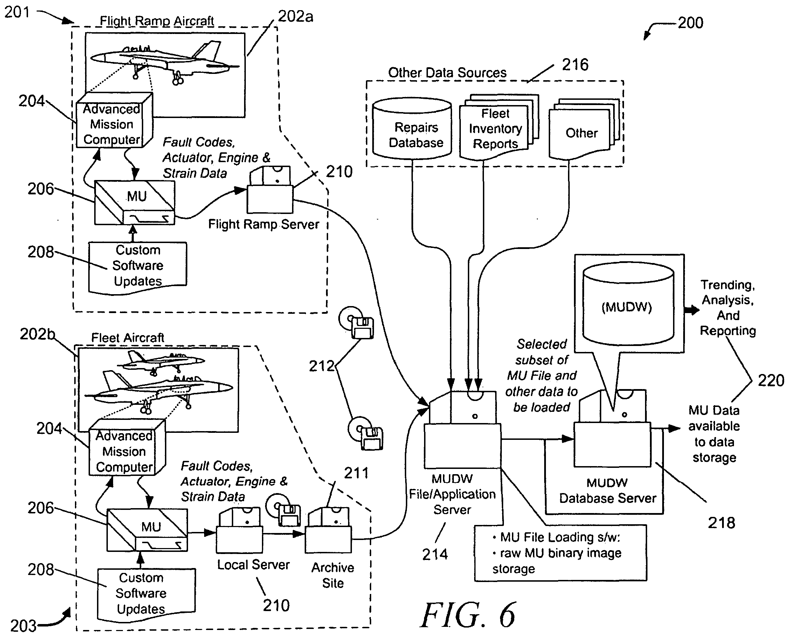

Figure 6 is another representative health data collection process 200. In this embodiment, the process 200 includes a first branch 201 that collects data from newly-built, pre-delivery aircraft 202a, and a second branch 203 that collects data from in-service aircraft 202b. The first and second branches 201, 203 have several components in common, and therefore, for the sake of brevity, the following description applies to both the first and second branches 201,203 unless otherwise stated. As shown in Figure 6, the military aircraft 202a, 202b include a mission computer 204 operatively coupled to a memory unit 206. A supplementary software program 208 is loaded to the memory unit 206 (and to the mission computer 204 during powerup). The supplementary software program 208 is configured to perform functions, such as suppressing known nuisance information, between updates to the mission computer 204.

Health data from the memory unit 206 are transmitted to a server 210, and in the second branch 203, to an archive 211 for storage. The health data are then transmitted (e.g. as data files 212) to a warehouse server 214 that also receives health data from other data sources 216. As depicted in Figure 10, access to all the data downloaded from the memory units may be provided, or alternately, if it is not practical to host all the data on a server due to the sheer volume of data that is contained on each memory unit, a selective subset of this data may be identified and retrieved. The selected sets or subsets of health data may be provided to a second warehouse server 218, and final health data 220 may be output from the second warehouse server 218 in a variety of formats using a variety of storage media for subsequent analysis using methods and systems as described above.

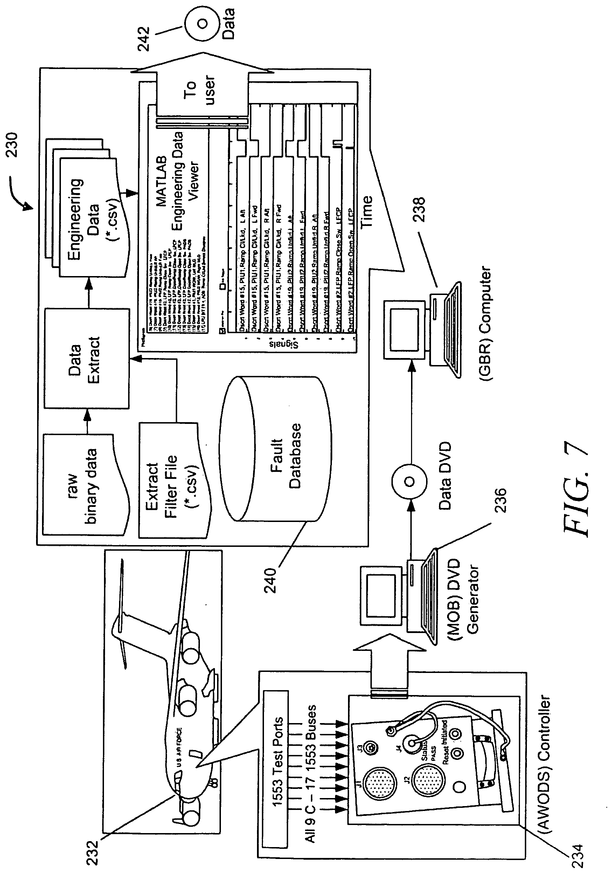

Figure 7 is another representative health data collection process 230 for another aircraft 232. In this embodiment, the aircraft 232 includes an on-board controller 234 that includes a data recorder. In a particular embodiment, the on-board controller 234 is an Advanced Wireless Open Data System (AWODS) installed in an avionics rack of the aircraft 232. The data recorder media or the on-board controller 234 is removed from the aircraft 232 and coupled to a server 236 which downloads the desired health data. One or more portions of the method 100 described above may be performed on the server 236 (*e.g.* translating and formatting the health data into a common format), or alternately, the health data may be transmitted to a fleet management server 238, which may perform one or more remaining portions of the method (or portions thereof) in accordance with the present disclosure. A fault database 240 is included in the fleet management server 238. The health data 242 from the on-board controller 234 may be stored in the fault database 240, and may be communicated to a health management system (*e.g.* system 120 described above) for further analysis.

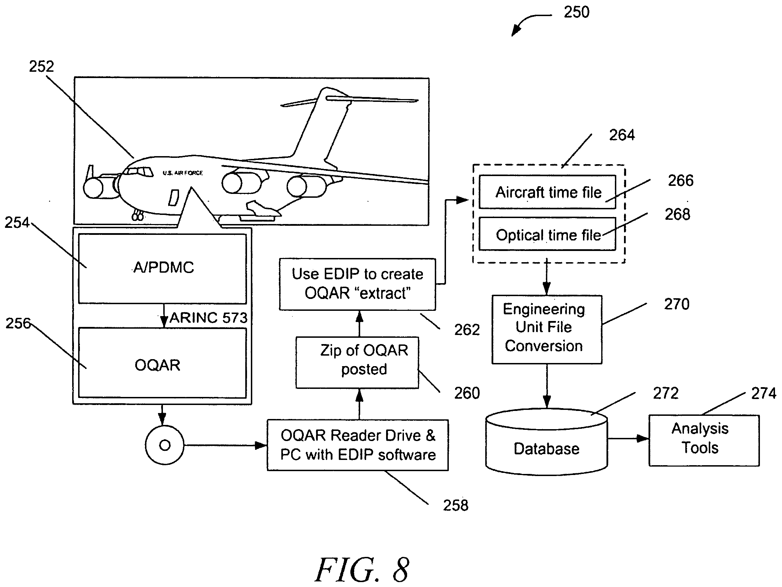

Figure 8 is still another representative health data collection process 250 for an aircraft 252 that includes a data management computer 254, and a recorder 256 coupled to the data management computer 254. In a particular embodiment, the recorder 256 may be an Optical Quick Access Recorder (OQAR). A server 258 receives health data from the recorder 256 and may output these data in a first format 260 (*e.g.* a Zip file). At an interim block 262, the health data may be modified, such as by extracting a portion of the data from the recorder 256 for further analysis. The resulting data are then received at an interface module 264, which may include an aircraft (A/C) time file 266, an optical time file 268, or other assorted files. The data from the interface module 264 may undergo a conversion at a bock 270, such as an engineering units conversion, prior to entry into a database 272. The health data may then be selectively accessed using analysis tools 274 for subsequent analysis and management.

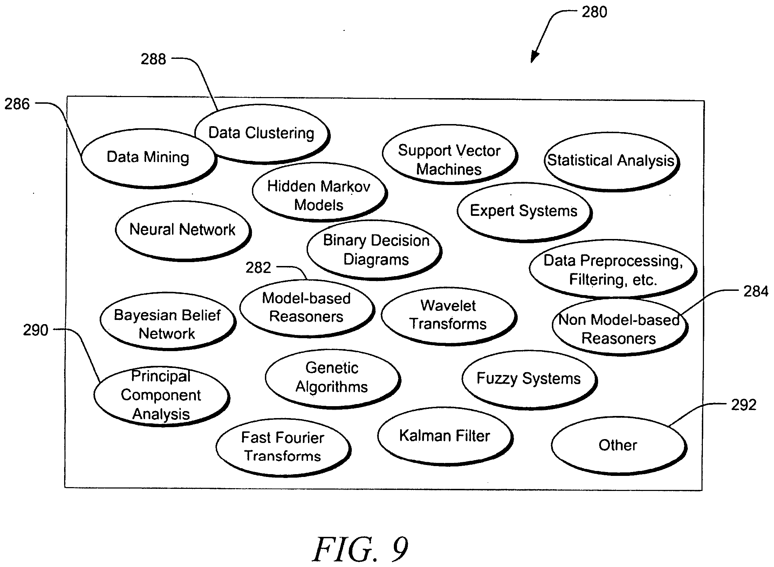

Figure 9 shows a plurality of representative diagnostic reasoning algorithms 280 suitable for use in systems and methods in accordance with alternate embodiments of the invention. The reasoning algorithms 280 include model based techniques 282 and non-model based techniques 284. More specifically, the reasoning algorithms 280 may include one or more data mining algorithms 286, data clustering 288 (*e.g*. data clustering using Trellis Diagram and Fisher Class Separability Measures), principal or independent component analysis (PCA or ICA) algorithms 290, , and any other suitable pre-processing or reasoning algorithms 292 such as clustered Self-Organizing Maps (SOM) algorithms, wavelet pre-processing of optimal data features algorithms, model-based fault detection and identification (ID) algorithms, interacting multi-model estimator algorithms, and hybrid probabilistic neural network (NN) classifiers.

From the foregoing description, it may be appreciated that embodiments of systems and methods in accordance with the present disclosure may advantageously provide an architectural framework, including data translation, storage, and diagnostic / prognostic analysis tools, to perform aircraft health assessments. Long-term benefits of such systems and methods may include: 1) development of technologies that address total ownership cost reduction, expeditionary logistics, and warfighter protection and enhanced safety, 2) reduced operating costs through life-extension of legacy systems and improved diagnostic tools to decrease the number of unnecessary parts removals, 3) improved affordability and safety throughout the commercial air transportation industry -- specifically, airline gate delay and air turnback/diversion costs will be reduced due to improved system health monitoring and prognostics, 4) additional cost reductions and safety improvement will result from new condition-based maintenance practices, 5) enable cost effective monitoring and assessment of existing aircraft data, 6) increase availability of warfighter assets resulting in reduced overall acquisition cost, 7) increase unmanned air vehicle (UAV) mission completion and increase survivability through Integrated Systems Health Management (ISHM) with reconfigurable control, and 8) more fully utilize the available data to achieve economic and safety objectives.

Various modules and techniques may be described herein in the general context of computer-executable instructions, such as program modules, executed by one or more computers or other devices. Generally, program modules include routines, programs, objects, components, data structures, and so forth for performing particular tasks. These program modules and the like may be executed as native code or may be downloaded and executed, such as in a virtual machine or other just-in-time compilation execution environment. Typically, the functionality of the program modules may be combined or distributed as desired in various embodiments. An implementation of these modules and techniques may be stored on or transmitted across some form of computer readable media.

While preferred and alternate embodiments of the invention have been illustrated and described, as noted above, many changes can be made without departing from the scope of the invention. Accordingly, the scope of the invention is not limited by the disclosure of these preferred and alternate embodiments. Instead, the invention should be determined entirely by reference to the claims that follow.

## Claims

1. A method of monitoring health information for a multi-platform system, comprising:
receiving health information from one or more subsystems of a plurality of platforms (104);
analyzing the health information using one or more reasoner algorithms (128) configured to at least one of diagnose and predict a potential failure of the one or more subsystems;
upon diagnosis of a failure, providing recommended action;
upon prediction of a potential failure, providing a prognostic characteristic of the one or more subsystems;
translating at least some of the health information for each of the one or more subsystems into a common format; and
storing the translated health information in a relational database (134) for subsequent analysis, wherein the relational database has access to high rate data files (130) stored externally to the relational database for specific event-driven analyses of specific data parameters.

2. The method of Claim 1, wherein the plurality of platforms include one or more flight vehicles, and wherein analyzing the health information includes:
retrieving the translated health information from the relational database; and
analyzing the translated health information using one or more ground-based reasoner algorithms.

3. The method of Claim 1, wherein providing a prognostic characteristic includes providing at least one of a repair order, a replacement order, and a maintenance order.

4. The method of Claim 1, wherein analyzing the health information includes analyzing the health information using the relational database.

5. The method of Claim 4, wherein analyzing the health information using the relational database includes analyzing the health information using the relational database that enables at least one of the following data associations: 1) multiple data set associated from the same data source, 2) two different data sources from the same aircraft, and 3) multiple different aircraft platforms.

6. A system for monitoring health information for a multi-platform system, comprising:
a first component (102) configured to receive health information from one or more subsystems of a plurality of platforms (104);
a second component (110) configured to analyze the health information using one or more reasoner algorithms (128) configured to at least one of diagnose and predict a potential failure of the one or more subsystems;
a third component (120) configured to, upon diagnosis of a failure, provide recommended action, and upon prediction of a potential failure, provide a prognostic characteristic of the one or more subsystems;
a fourth component (122) configured to translate at least some of the health information for each of the one or more subsystems into a common format; and
a fifth component (126) configured to store the translated health information in a relational database (134) for subsequent analysis, wherein the relational database has access to high rate data files (130) stored externally to the relational database for specific event-driven analyses of specific data parameters.

7. The system of Claim 6, wherein the plurality of platforms include one or more flight vehicles, and wherein the second component is further configured to:
retrieve the translated health information from the relational database; and
analyze the translated health information using one or more ground-based reasoner algorithms (128).

8. The system of Claim 6, wherein the third component is further configured to provide at least one of a repair order, a replacement order, and a maintenance order.

9. The system of Claim 6, wherein the second component is further configured to analyze the health information using the relational database.

10. The system of Claim 6, wherein the second component is further configured to analyze the health information using the relational database that enables at least one of the following data associations: 1) multiple data set associated from the same data source, 2) two different data sources from the same aircraft, and 3) multiple different aircraft platforms.

11. One or more computer-readable media containing computer-readable instructions that, when executed, perform a method of monitoring health information for a multi-platform system, comprising:
receiving health information from one or more subsystems of a plurality of platforms (104);
analyzing the health information using one or more reasoner algorithms (128) configured to at least one of diagnose and predict a potential failure of the one or more subsystems;
upon diagnosis of a failure, providing recommended action;
upon prediction of a potential failure, providing a prognostic characteristic of the one or more subsystems;
translating at least some of the health information for each of the one or more subsystems into a common format; and
storing the translated health information in a relational database (134) for subsequent analysis, wherein the relational database has access to high rate data files (130) stored externally to the relational database for specific event-driven analyses of specific data parameters.

12. The computer-readable media of Claim 11, wherein the plurality of platforms include one or more flight vehicles, and wherein analyzing the health information includes:
retrieving the translated health information from the relational database; and
analyzing the translated health information using one or more ground-based reasoner algorithms.

## Patentansprüche

1. Verfahren zum Überwachen von Integritätsinformationen für ein Multiplattformsystem, das Folgendes aufweist:
Empfangen von Integritätsinformationen von einem oder mehreren Untersystemen mehrerer Plattformen (104);
Analysieren der Integritätsinformationen unter Verwendung eines oder mehrerer Algorithmen (128) eines Reasoners, die konfiguriert sind, um mindestens eine mögliche Fehlfunktion des einen oder der mehreren Untersysteme zu diagnostizieren oder vorherzusagen;
bei Feststellung einer Fehlfunktion das Bereitstellen einer empfohlenen Aktion;
bei Vorhersage einer potenziellen Fehlfunktion das Bereitstellen eines prognostischen Merkmals des einen oder der mehreren Untersysteme;
Übersetzen mindestens einiger der Integritätsinformationen für jedes des einen oder der mehreren Untersysteme in ein einheitliches Format; und
Speichern der übersetzten Integritätsinformationen in einer relationalen Datenbank (134) zur anschließenden Analyse, wobei die relationale Datenbank auf Datendateien (130) mit hoher Rate, die außerhalb der relationalen Datenbank für spezifische ereignisgesteuerte Analysen spezifischer Datenparameter gespeichert sind, Zugriff hat.

2. Verfahren nach Anspruch 1, wobei die mehreren Plattformen ein oder mehrere Fluggeräte enthalten und wobei das Analysieren der Integritätsinformationen Folgendes enthält:
Abrufen der übersetzten Integritätsinformationen aus der relationalen Datenbank; und
Analysieren der übersetzten Integritätsinformationen unter Verwendung eines oder mehrerer bodengestützter Algorithmen eines Reasoners.

3. Verfahren nach Anspruch 1, wobei das Bereitstellen eines prognostischen Merkmals das Bereitstellen von mindestens einem von einem Reparaturauftrag, einem Austauschauftrag und einem Instandhaltungsauftrag enthält.

4. Verfahren nach Anspruch 1, wobei das Analysieren der Integritätsinformationen das Analysieren der Integritätsinformationen unter Verwendung der relationalen Datenbank enthält.

5. Verfahren nach Anspruch 4, wobei das Analysieren der Integritätsinformationen unter Verwendung der relationalen Datenbank das Analysieren der Integritätsinformationen unter Verwendung der relationalen Datenbank enthält, die mindestens eine der folgenden Datenzuordnungen ermöglicht: 1) vielfache Datensätze, die derselben Datenquelle zugeordnet sind, 2) zwei unterschiedliche Datenquellen von demselben Luftfahrzeug und 3) vielfache unterschiedliche Luftfahrzeugplattformen.

6. System zum Überwachen von Integritätsinformationen für ein Multiplattformsystem, das Folgendes aufweist:
eine erste Komponente (102), die konfiguriert ist, um Integritätsinformationen aus einem oder mehreren Untersystemen mehrerer Plattformen (104)zu empfangen;
eine zweite Komponente (110), die konfiguriert ist, um Integritätsinformationen unter Verwendung eines oder mehrerer Algorithmen (128) eines Reasoners, die konfiguriert sind, um mindestens eine mögliche Fehlfunktion des einen oder der mehreren Untersysteme zu diagnostizieren oder vorherzusagen, zu analysieren;
eine dritte Komponente (120), die konfiguriert ist, um bei Feststellung einer Fehlfunktion eine empfohlene Aktion bereitzustellen und bei Vorhersage einer potenziellen Fehlfunktion ein prognostisches Merkmal des einen oder der mehreren Untersysteme bereitzustellen;
eine vierte Komponente (122), die konfiguriert ist, um mindestens einige der Integritätsinformationen für jedes des einen oder der mehreren Untersysteme in ein einheitliches Format zu übersetzen; und
eine fünfte Komponente (126), die konfiguriert ist, um die übersetzten Integritätsinformationen in einer relationalen Datenbank (134) für die anschließende Analyse zu speichern, wobei die relationale Datenbank auf Datendateien (130) mit hoher Rate, die außerhalb der relationalen Datenbank für spezifische ereignisgesteuerte Analysen von spezifischen Datenparametern gespeichert sind, Zugriff hat.

7. System nach Anspruch 6, wobei die mehreren Plattformen ein oder mehrere Fluggeräte enthalten und wobei die zweite Komponente ferner konfiguriert ist, um:
die übersetzten Integritätsinformationen aus der relationalen Datenbank abzurufen; und
die übersetzten Integritätsinformationen unter Verwendung eines oder mehrerer bodengestützter Algorithmen (128) eines Reasoners zu analysieren.

8. System nach Anspruch 6, wobei die dritte Komponente ferner konfiguriert ist, um mindestens eines von einem Reparaturauftrag, einem Austauschauftrag und einem Instandhaltungsauftrag bereitzustellen.

9. System nach Anspruch 6, wobei die zweite Komponente ferner konfiguriert ist, um die Integritätsinformationen unter Verwendung der relationalen Datenbank zu analysieren.

10. System nach Anspruch 6, wobei die zweite Komponente ferner konfiguriert ist, um die Integritätsinformationen unter Verwendung der relationalen Datenbank zu analysieren, die mindestens eine der folgenden Datenzuordnungen ermöglicht: 1) vielfachen Datensätze, die derselben Datenquelle zugeordnet sind, 2) zwei unterschiedliche Datenquellen von demselben Luftfahrzeug und 3) vielfache unterschiedliche Luftfahrzeugplattformen.

11. Ein oder mehrere computerlesbare Medien, die computerlesbare Anweisungen enthalten, die, wenn sie ausgeführt werden, ein Verfahren zum Überwachen von Integritätsinformationen für ein Multiplattformsystem durchführen, Folgendes aufweisend:
Empfangen von Integritätsinformationen von einem oder mehreren Untersystemen mehrerer Plattformen (104);
Analysieren der Integritätsinformationen unter Verwendung eines oder mehrerer Algorithmen (128) eines Reasoners, die konfiguriert sind, um mindestens eine mögliche Fehlfunktion des einen oder der mehreren Untersysteme zu diagnostizieren oder vorherzusagen;
bei Feststellung einer Fehlfunktion das Bereitstellen empfohlener Aktion;
bei Vorhersage einer möglichen Fehlfunktion das Bereitstellen eines prognostischen Merkmals des einen oder der mehreren Untersysteme;
Übersetzen mindestens einiger der Integritätsinformationen für jedes des einen oder der mehreren Untersysteme in ein einheitliches Format; und
Speichern der übersetzten Integritätsinformationen in einer relationalen Datenbank (134) zur anschließenden Analyse, wobei die relationale Datenbank auf Datendateien (130) mit hoher Rate, die außerhalb der relationalen Datenbank für spezifische ereignisgesteuerte Analysen von spezifischen Datenparametern gespeichert sind, Zugriff hat.

12. Computerlesbare Medien nach Anspruch 11, wobei die mehreren Plattformen ein oder mehrere Fluggeräte enthalten und wobei das Analysieren der Integritätsinformationen Folgendes enthält:
Abrufen der übersetzten Integritätsinformationen von der relationalen Datenbank; und
Analysieren der übersetzten Integritätsinformationen unter Verwendung eines oder mehrerer bodengestützter Algorithmen eines Reasoners.

## Revendications

1. Procédé de contrôle d'informations de bon état pour un système multi-plateforme, comprenant :
la réception d'informations de bon état depuis un ou plusieurs sous-systèmes d'une pluralité de plateformes (104) ; ;
l'analyse des informations de bon état en utilisant au moins un algorithme *reasoner* (128) conçu pour au moins diagnostiquer et prédire un risque de défaillance d'un ou plusieurs sous-systèmes ;
en cas de diagnostic d'une défaillance, la fourniture d'une action recommandée ;
en cas de prédiction d'un risque de défaillance, la fourniture d'une caractéristique pronostique d'un ou plusieurs sous-systèmes ;
la traduction d'au moins certaines des informations de bon état pour chacun des au moins un sous-systèmes en un format commun ; et
l'enregistrement des informations de bon état traduites dans une base de données relationnelle (134) pour analyse ultérieure, la base de données relationnelle ayant accès à des fichiers de données haut débit (130) enregistrés en externe à la base de données relationnelle pour des analyses spécifiques en vue d'analyses spécifiques fonctions d'événements de paramètres de données spécifiques.

2. Procédé selon la revendication 1, dans lequel la pluralité de plateformes inclut au moins un véhicule aérien, et dans lequel les informations de bon état incluent :
l'extraction des informations de bon état traduites de la base de données relationnelle ; et
l'analyse des informations de bon état traduites en utilisant un ou plusieurs algorithmes *reasoners* de terrain.

3. Procédé selon la revendication 1, dans lequel la fourniture d'une caractéristique pronostique inclut au moins un parmi un ordre de réparation, un ordre de remplacement et un ordre de maintenance.

4. Procédé selon la revendication 1, dans lequel l'analyse des informations de bon état inclut l'analyse des informations de bon état en utilisant la base de données relationnelle.

5. Procédé selon la revendication 4, dans lequel l'analyse des informations de bon état inclut l'analyse des informations de bon état en utilisant la base de données relationnelle qui permet au moins une des associations de données suivantes :
1) de multiples ensembles de données associées depuis la même source de données,
2) deux différentes source de données depuis le même avion, et
3) de multiples plateformes aériennes différentes.

6. Système de contrôle d'informations de bon état pour un système multi-plateforme, comprenant :
un premier composant (102) conçu pour recevoir des informations de bon état depuis un ou plusieurs sous-systèmes d'une pluralité de plateformes (104) ;
un deuxième composant (110) conçu pour analyser les informations de bon état en utilisant un ou plusieurs algorithmes *reasoner* (128) conçus pour au moins un parmi le diagnostic et la prédiction d'un risque de défaillance d'un ou plusieurs sous-systèmes ;
un troisième composant (120) conçu pour, en cas de diagnostic de défaillance, fournir une action recommandée, et en cas de diagnostic de risque de défaillance, fournir une caractéristique diagnostique d'au moins un sous-système ;
un quatrième composant (122) conçu pour traduire au moins certaines des informations de bon état pour chacun des au moins un sous-système en un format commun ;
un cinquième composant (126) conçu pour enregistrer les informations de bon état traduites dans une base de données relationnelle (134) pour analyse ultérieure, la base de données relationnelle ayant accès à des fichiers de données haut débit (130) enregistrés en externe à la base de données relationnelle pour des analyses spécifiques en vue d'analyses spécifiques fonctions d'évènements de paramètres de données spécifiques.

7. Système selon la revendication 6, dans lequel la pluralité de plateformes inclut au moins un véhicule aérien, et dans lequel le deuxième composant est conçu en outre pour :
extraire des informations de bon état traduites de la base de données relationnelle ; et
analyser des informations de bon état traduites en utilisant un ou plusieurs algorithmes *reasoners* de terrain (128).

8. Système selon la revendication 6, dans lequel le troisième composant est conçu pour fournir au moins un parmi un ordre de réparation, un ordre de remplacement et un ordre de maintenance.

9. Système selon la revendication 6, dans lequel le deuxième composant est conçu en outre pour analyser les informations de bon état en utilisant la base de données relationnelle.

10. Système selon la revendication 6, dans lequel le deuxième composant est conçu en outre pour analyser les informations de bon état en utilisant la base de données relationnelle qui permet au moins une parmi les associations de données suivantes :
1) de multiples ensembles de données associées depuis la même source de données,
2) deux différentes source de données depuis le même avion, et
3) de multiples plateformes aériennes différentes.

11. Au moins un support lisible par un ordinateur contenant des instructions lisibles par un ordinateur et qui, quand on les exécute, effectue un procédé de contrôle d'informations de bon état pour un système multiplateforme, comprenant :
la réception la réception d'informations de bon état depuis un ou plusieurs sous-systèmes d'une pluralité de plateformes (104) ;
l'analyse des informations de bon état en utilisant au moins un algorithme *reasoner* (128) conçu pour au moins diagnostiquer et prédire un risque de défaillance d'un ou plusieurs sous-systèmes ;
en cas de diagnostic d'une défaillance, la fourniture d'une action recommandée ;
en cas de prédiction d'un risque de défaillance, la fourniture d'une caractéristique pronostique d'un ou plusieurs sous-systèmes ;
la traduction d'au moins certaines des informations de bon état pour chacun des au moins un sous-systèmes en un format commun ; et
l'enregistrement des informations de bon état traduites dans une base de données relationnelle (134) pour analyse ultérieure, la base de données relationnelle ayant accès à des fichiers de données haut débit (130) enregistrés en externe à la base de données relationnelle pour des analyses spécifiques en vue d'analyses spécifiques fonctions d'évènements de paramètres de données spécifiques.

12. Support lisible par un ordinateur selon la revendication 11, dans lequel la pluralité de plateformes inclut au moins un véhicule aérien, et dans lequel l'analyse des informations de bon état inclut :
l'extraction des informations de bon état traduites de la base de données relationnelle ; et
l'analyse des informations de bon état traduites en utilisant un ou plusieurs algorithmes *reasoners* de terrain.
